# FASCICULE DE BREVET EUROPEEN

(11) **EP 2 293 785 B1**
(45) Date de publication et mention de la délivrance du brevet: **11.07.2012**
(21) Numéro de dépôt: 09761922.5
(22) Date de dépôt: 20.05.2009
(51) Int. Cl.: A61K 9/51, A61K 38/17, A61P 37/00, A61K 47/36

(54) **NANOPARTICULES CONTENANT UN PEPTIDE, VECTEURS LES CONTENANT ET UTILISATIONS PHARMACEUTIQUES DESDITS NANOPARTICULES ET VECTEURS**
EIN PEPTID ENTHALTENDE NANOTEILCHEN; DIESE ENTHALTENDE VEKTOREN UND PHARMAZEUTISCHE ANWENDUNGEN DIESER NANOTEILCHEN UND VEKTOREN
NANOPARTICLES CONTAINING A PEPTIDE, VECTORS CONTAINING THE SAME AND PHARMACEUTICAL USES OF SAID NANOPARTICLES AND SAID VECTORS

(30) Priorité: 20.05.2008 FR 0853264
(43) Date de publication de la demande: 16.03.2011
(73) Titulaire: Centre National de la Recherche Scientifique (C.N.R.S), 75016 Paris (FR); Université de Strasbourg, 67081 Strasbourg Cedex (FR)
(72) Inventeur: DANICHER, Louis, F-67200 Strasbourg (FR); FRERE, Yves, F-67810 Holtzheim (FR); MULLER, Sylviane, F-67000 Strasbourg (FR); WAWREZINIECK, Anne, F-44700 Orvault (FR)
(74) Mandataire: Blot, Philippe Robert Emile
(86) Numéro de dépôt international: PCT/FR2009/050944
(87) Numéro de publication internationale: WO 2009/150371

(56) Documents cités:
- WO-A-03/020747
- WO-A-03/025014
- MONNEAUX F ET AL: "T CELL RECOGNITION AND THERAPEUTIC EFFECT OF A PHOSPHORYLATED SYNTHETIC PEPTIDE OF THE 70K SNRNP PROTEIN ADMINISTERED IN MRL/LPR MICE" EUROPEAN JOURNAL OF IMMUNOLOGY, WILEY - V C H VERLAG GMBH & CO. KGAA, DE, vol. 33, no. 2, 1 février 2003 (2003-02-01), pages 287-296, XP001148289 ISSN: 0014-2980 cité dans la demande
- PAULETTI G M ET AL: "Improvement of oral peptide bioavailability: Peptidomimetics and prodrug strategies" ADVANCED DRUG DELIVERY REVIEWS, ELSEVIER BV, AMSTERDAM, NL, vol. 27, no. 2-3, 1 janvier 1997 (1997-01-01), pages 235-256, XP002234351 ISSN: 0169-409X

### SEQUENCE LISTING

<110> CNRS
   université Louis Pasteur
<120> Nanoparticules contenant un peptide, vecteurs les contenant et utilisations pharmaceutiques desdits nanoparticules et vecteurs
<130> BFF 07P0605
<160> 1
<170> PatentIn version 3.4
<210> 1
   <211> 21
   <212> PRT
   <213> Homo sapiens
<220>
   <221> MOD_RES
   <222> (10)..(10)
   <223> PHOSPHORYLATION
<400> 1
   Arg Ile His Met Val Tyr Ser Lys Arg Ser Gly Lys Pro Arg Gly Tyr
   1               5                   10                  15
   Ala Phe Ile Glu Tyr
               20

## Description

La présente invention a pour objet des nanoparticules contenant un peptide, ainsi que des véhicules, avec ou sans dispersant, contenant lesdites nanoparticules. La présente invention a également pour objet des utilisations pharmaceutiques et/ou thérapeutiques desdits vecteurs, notamment pour le traitement du lupus érythémateux disséminé.

Le lupus érythémateux disséminé (LED) est une maladie auto-immune inflammatoire chronique très invalidante qui affecte aujourd'hui plusieurs millions de personnes dans le monde. Cette maladie résulte d'un dysfonctionnement du système immunitaire qui reconnaît les éléments du soi comme étrangers et développe alors une réponse immune dont une production d'anticorps contre les propres constituants de l'organisme.

Une voie thérapeutique prometteuse est basée sur l'utilisation de séquences peptidiques dérivées d'auto-antigènes qui peuvent moduler la réponse auto-immune en interférant avec la production des auto-anticorps. A cet égard, la demande internationale WO 03/025014 décrit l'utilisation de peptides spécifiques dans le cadre du traitement du lupus érythémateux disséminé. Plus particulièrement, cette demande internationale décrit l'utilisation du peptide P140, qui est un analogue phosphorylé sur le résidu sérine en position 140 du peptide 131-151 de la protéine splicéosomale U1-70K (protéine spécifique de la particule U1-snRNP), notamment dans le cadre du traitement du lupus érythémateux disséminé.

A ce jour, le peptide P140 est administré par injection sous-cutanée, qui n'est pas un mode d'administration confortable pour le patient. Ainsi, plusieurs autres modes d'administration ont été testés. Ainsi, l'administration intranasale a été testée chez la souris mais n'est pas efficace.

Dans le domaine thérapeutique, les voies d'administration de principes actifs les plus courantes sont les voies orale, intraveineuse, intramusculaire ou sous-cutanée. Cependant, la voie orale, qui est la plus physiologique et la plus confortable pour le patient, ne peut être utilisée pour de nombreuses substances actives comme les peptides ou les protéines. En effet, ces macromolécules de type peptidique ou protéique subissent de nombreuses agressions le long du tractus gastro-intestinal qui altèrent leur structure et leur fonction biologique. Par ailleurs, leur passage à travers la paroi intestinale est limité par leur taille et leur caractère hydrophile. Tout ceci explique la faible biodisponibilité des protéines administrées par voie orale (de l'ordre de 1 à 2%).

Ainsi, un des buts de la présente invention consiste à améliorer l'efficacité du peptide P140 susmentionné dans le cadre d'une administration par voie orale.

Un autre but de la présente invention consiste à fournir le peptide P140 sous forme encapsulée, administrable par voie orale, et permettant d'atteindre le même type de cellules avec la même efficacité que lorsque le peptide est administré par voie sous-cutanée.

La présente invention a donc pour but de fournir un système d'administration par voie orale du peptide P140 susmentionné possédant des caractéristiques de disponibilité dudit peptide comparables aux caractéristiques d'injection directe dans le sang dudit peptide.

Un autre but de l'invention consiste à fournir un système d'administration permettant la libération dudit peptide qui soit peu ou pas dénaturé ou dégradé lors d'une administration par voie orale.

Un autre but de l'invention consiste à fournir un système d'administration permettant audit peptide d'atteindre et/ou de traverser la paroi intestinale sans être dénaturé ou dégradé substantiellement.

Un autre but de l'invention consiste à fournir un système d'administration permettant audit peptide ayant traversé la paroi intestinale d'être disponible dans le sang, éventuellement après passage dans le liquide interstitiel.

Un autre but de l'invention consiste à fournir un système d'administration par voie orale du peptide P140 dans lequel ledit peptide n'est pas ou peu dénaturé ou dégradé lors du passage dans le tractus gastro-intestinal et lors du passage de la paroi intestinale, et qui permette une disponibilité immédiate, retardée ou prolongée dans le sang.

La présente invention concerne des nanoparticules comprenant une matrice constituée d'au moins un polysaccharide et du peptide P140 de séquence SEQ ID NO : 1 ou d'un de ses analogues, ledit polysaccharide étant de préférence un polysaccharide porteur de groupement(s) fonctionnel(s) négativement chargé(s).

Le peptide P140 est un peptide de 21 résidus d'acides aminés d'une masse molaire d e 2 636 Da qui correspond à la séquence suivante : RIHMVYSKRSGKPRGYAFIEY, dans laquelle la sérine en position 10 est phosphorylée (SEQ ID NO : 1).

L'expression "analogues du peptide P140" désigne des peptides comprenant la séquence SEQ ID NO: 1 susmentionnée dans laquelle l'un au moins des acides aminés comporte une modification de type post-traductionnel, notamment par phosphorylation ou acétylation. Parmi les analogues, on peut faire référence aux peptides décrits dans la demande internationale WO 03/025014.

L'expression "groupement(s) fonctionnel(s) négativement chargé(s)" désigne des fonctions chimiques telles que par exemple les groupements carboxyliques.

Le polysaccharide constituant la matrice doit être biocompatible, biodégradable et bioassimilable (métabolisable).

Selon un autre mode de réalisation, les nanoparticules selon l'invention peuvent être préparées à partir d'une matrice constituée d'un mélange de polysaccharides et du peptide P140. Plus particulièrement, ces nanoparticules peuvent comprendre une matrice constituée du peptide P140 et d'un mélange d'acide hyaluronique et de chitosane.

Selon un mode particulièrement avantageux, les nanoparticules selon l'invention sont obtenues par coacervation complexe entre le peptide et le polysaccharide.

Selon un mode de réalisation avantageux, les nanoparticules selon la présente invention comprennent une matrice constituée d'acide hyaluronique.

L'acide hyaluronique est non-toxique, biocompatible et métabolisable puisque présent dans de nombreux tissus.

Dans ce mode de réalisation, les nanoparticules sont obtenues par coacervation complexe entre le peptide P140 et l'acide hyaluronique.

De préférence, la taille des nanoparticules selon l'invention est comprise d'environ 1 nm à environ 10 µm.

Selon un mode de réalisation particulièrement avantageux, dans les nanoparticules selon la présente invention, le rapport massique entre le polysaccharide et le peptide est compris d'environ 0,1 à environ 10, et notamment compris d'environ 0,5 à environ 5, et de préférence égal à environ 1.

Ce rapport massique influe sur le rapport entre les charges positives et négatives présentes dans la solution.

Il a été constaté que la valeur de ce rapport massique influe sur la taille des nanoparticules obtenues.

De préférence, lorsque le polysaccharide est l'acide hyaluronique, ce rapport est égal à environ 1 (1,5 charge négative pour 1 charge positive), ce qui permet alors d'assurer la stabilité colloïdale. Lorsque ce rapport est égal à 1, on obtient alors des nanoparticules sphériques d'un diamètre proche de 200 nm et de charge de surface négative (ξ = -30 mV), qui peuvent être aisément centrifugées et qui sont stables pendant plusieurs semaines à 4°C.

Les nanoparticules préférées selon la présente invention sont des nanoparticules telles que définies ci-dessus dans lesquelles la masse molaire du polysaccharide est comprise d'environ 1 000 Da à environ 1 500 000 Da, et de préférence d'environ 15 000 Da à environ 50 000 Da, et notamment d'environ 20 000 Da à environ 30 000 Da.

De préférence, les nanoparticules sont préparées à partir d'acide hyaluronique de masse molaire égale à 27600 Da. Dans ce cas, les nanoparticules obtenues ont une taille proche de 200 nm et sont parfaitement sphériques. Elles sont alors également très monodisperses avec un indice de polydispersité inférieur à 0,1. Ces particules peuvent être isolées du milieu de synthèse par centrifugation à une vitesse de 2 370G sans problème d'agrégation. Elles présentent une charge de surface négative avec un potentiel zêta de l'ordre de -30 mV. Leur surface est majoritairement recouverte d'acide hyaluronique et le peptide P140, de charge positive, condensé à l'intérieur, est protégé de l'environnement extérieur par ce polyélectrolyte. Cette charge de surface assure une bonne stabilité de la suspension au stockage (au moins deux semaines à 4°C) et à la centrifugation.

La présente invention concerne également un vecteur complexe pour administration par voie orale constitué d'un véhicule qui comprend au moins une nanoparticule telle que définie ci-dessus.

La nanoparticule et le véhicule sont deux vecteurs, le premier ayant pour but de faire franchir la barrière intestinale au peptide, le second de faire franchir le milieu gastrique à la nanoparticule. L'ensemble de ces deux vecteurs forme un vecteur complexe.

L'administration du peptide P140 (ou analogue) par voie orale consiste en fait à apporter, de la bouche vers le sang ou les tissus, ledit peptide, sans que celui-ci soit substantiellement dénaturé ou dégradé. On entend ainsi que la dose de peptide P140 (ou analogue) administrée par voie orale doit pouvoir se retrouver de manière substantiellement qualitative et quantitative dans le sang ou les tissus. Par "substantiellement quantitative et qualitative", on entend que la quantité de peptide P140 (ou analogue) dans le sang ou les tissus par rapport à la quantité de P140 (ou analogue) administrée par voie orale doit être supérieure et avantageusement à au moins 20%, notamment supérieure à 50%, de préférence supérieure à 65%, avantageusement supérieure à 80%, de manière optimale, supérieure à 90%.

De préférence, la nanoparticule contenue dans le vecteur complexe tel que défini ci-dessus est bioassimilable ou métabolisable à un pH compris d'environ 6 à environ 8, notamment d'environ 6,7 à environ 7,7, et de préférence d'environ 7,2 à environ 7,5.

Lors d'une administration par voie orale, la substance active du point de vue pharmacologique (peptide P140 ou analogue) doit franchir tous les obstacles présents dans un organisme avant de parvenir dans le flux sanguin ou les tissus, et ce, sans subir de dénaturations ou de dégradations substantielles. Les principaux obstacles et difficultés, pris en compte dans le cadre de la présente invention, sont tout d'abord le passage de la substance active dans l'estomac, le séjour dans la lumière intestinale, l'adhésion aux microvillosités présentes dans l'intestin, le passage de l'intestin vers le sang, éventuellement via le liquide interstitiel.

La substance active (peptide P140 ou analogue) administrée par voie orale doit parvenir sans dénaturation ou dégradation substantielle dans le sang ou dans le liquide interstitiel ou les tissus.

En considérant que les caractéristiques du sang et du milieu interstitiel sont différentes des autres organes (estomac, intestin entre autres) traversés depuis la cavité buccale, les inventeurs ont imaginé un vecteur pour le peptide P140 (ou analogue), vecteur qui puisse être biocompatible et bioassimilable ou métabolisable.

Ainsi, ce vecteur doit être hydrophile, pour être compatible avec les divers fluides corporels (liquide lymphatique, liquide interstitiel, sang, ...). Le vecteur doit en outre libérer rapidement, ou de manière prolongée ou de manière retardée, le peptide P140 à un pH compris entre environ 6,7 et 7,7, idéalement entre environ 7,2 et 7,5, pour permettre au peptide P140 d'être ensuite disponible dans le sang, à travers le vecteur et/ou après dégradation de celui-ci.

Sans entrer dans des considérations mécanistiques complexes, il est envisagé que le vecteur soit dégradé par les enzymes présentes dans le milieu (lysozyme, estérases, glycosidases, ...). La dégradation du vecteur permettra la libération immédiate, prolongée ou retardée du peptide P140 dans le liquide interstitiel pour atteindre la circulation sanguine ou les tissus. Une fois dans le sang, le peptide P140 interagira avec les sites d'intérêt, ou sera transporté jusqu'aux sites ou organes, afin de produire l'effet pharmacologique désiré.

De plus, ce vecteur doit être optimisé de sorte que sa nature hydrophile soit modifiée afin de la rendre compatible avec la paroi de l'intestin. En effet, la paroi de l'intestin recouverte d'un mucus est un milieu essentiellement lipophile dont le pH est supérieur à environ 7,8. Il convient par conséquent de modifier le vecteur décrit ci-dessus de sorte qu'il soit essentiellement lipophile au voisinage et sur la paroi intestinale, qu'il présente une muco-adhésion relativement bonne et enfin qu'il résiste à l'environnement entérique (milieu basique de pH supérieur à environ 7,8, présence d'enzymes de dégradation, ...).

Selon un mode de réalisation avantageux, le vecteur complexe selon la présente invention a sa plus grande dimension comprise d'environ 10 nm à environ 2,5 cm, de préférence d'environ 100 nm à environ 1 cm, et notamment d'environ 0,5 mm à environ 2 mm.

Il convient en outre que le vecteur (nanoparticule) présent dans la lumière de l'intestin possède une taille et une forme permettant le passage physique dudit vecteur au travers de la membrane intestinale.

Une taille inférieure à environ 10 nm est également moins préférée, la quantité de substance active transportée par le vecteur pouvant être trop faible. En effet, la taille du vecteur nanoparticule est limitée par le lieu de passage de la nanoparticule (voie paracellulaire, voie transcellulaire, voie lymphatique).

La présente invention concerne également un vecteur complexe tel que défini ci-dessus, dans lequel le véhicule se présente sous forme de sphères de diamètre compris d'environ 10 nm à environ 2,5 cm, de préférence d'environ 100 nm à environ 1 cm, et notamment d'environ 0,5 mm à environ 2 mm.

La forme du vecteur (nanoparticule) n'a pas d'importance spécifique en soi pour autant qu'elle permette un passage aisé au travers de la paroi intestinale. Ainsi, le vecteur pourra se présenter sous toute forme connue, par exemple sphère, spaghetti, ovoïde, etc...

Lorsque le vecteur complexe se présente sous forme de sphères, celui-ci peut être préparé selon les techniques classiques d'encapsulation de substances actives, telles que, par exemple, par coacervation, simple ou complexe, polycondensation interfaciale, nébulisation/séchage (spray-drying), enrobage en lit fluidisé (spray-coating), etc...

Le vecteur (nanoparticule) selon la présente invention comprend une matrice renfermant le peptide P140. À ce titre, la matrice peut être conçue sous la forme d'un gel renfermant ledit peptide ou l'un de ses analogues. Selon un autre aspect, la matrice se présente sous forme d'une sphère renfermant ledit peptide ou l'un de ses analogues. D'autres formes encore sont envisageables, par exemple des formes de type "éponge", ou toutes autres formes solides plus ou moins compactes et pouvant libérer par diffusion et/ou après dégradation, la ou les substances actives (peptide P140 ou l'un des ses analogues) qu'elles renferment.

Il doit être précisé que, outre la ou les substances actives, le vecteur (nanoparticule) peut également contenir tout excipient, charge, colorant, et autres appropriés, connus de l'homme du métier, et non toxiques du point de vue pharmacologique.

Selon un mode de réalisation particulièrement avantageux, le vecteur complexe selon l'invention comprend un véhicule destiné à une protection gastrique.

Pour être efficace sur le plan pharmacologique, ce vecteur complexe destiné à être administré par voie orale doit en outre présenter une forte résistance au milieu stomacal par lequel il va transiter avant de parvenir à l'intestin. L'estomac est en effet un organe dans lequel le pH est très acide (aux alentours de 2, voire inférieur). De plus les enzymes présentes (en particulier la pepsine) dans l'estomac peuvent dénaturer, endommager voire détruire complètement ledit vecteur et par là même la ou les substances actives qu'il renferme.

Par conséquent, il est souhaitable de fournir une protection gastrique au vecteur défini précédemment. Par protection gastrique du vecteur, on entend tout véhicule capable de protéger ledit vecteur des contraintes physiologiques inhérentes à l'estomac, ces contraintes étant principalement le pH acide et les enzymes stomacales (pepsine). Bien entendu, les constituants du véhicule, ainsi que leurs produits de dénaturation ou de dégradation doivent être non toxiques pour l'organisme et biotoléré.

De tels véhicules sont déjà très largement connus dans le domaine (médicaments encapsulés par exemple, comme décrit dans "Encyclopedia of Pharmaceutical Technology", Marcel Dekker, (1992), J. Swarbrick and J. C. Boylan Editors, Enteric Coatings, pp. 189-200). Tout véhicule gastro-résistant connu de l'homme du métier peut par conséquent être utilisé. De préférence, il peut être de nature solide, sous forme de gel, ou se présenter sous la forme d'un revêtement ou d'une capsule, et renfermer un ou plusieurs vecteurs tels que définis précédemment, eux-mêmes sous diverses formes, capsules, gels ou autres.

Selon un aspect préféré de la présente invention, le véhicule gastro-résistant se présente sous la forme d'une capsule contenant un ou plusieurs vecteurs tels que définis précédemment. Lesdits véhicules sous forme de capsule peuvent avantageusement être obtenus par des procédés de type coacervation, polycondensation interfaciale en milieu dispersé, ou autres. Bien entendu, tout autre procédé connu d'encapsulation peut être utilisé et/ou adapté en vue de préparer les véhicules de l'invention.

Parmi les constituants capables de résister aux contraintes physiologiques inhérentes à l'estomac, on peut citer en particulier les alginates, comme l'alginate de calcium, la carboxyméthylcellulose, et autres, ainsi que leurs mélanges. Le véhicule gastro-protecteur devra résister à un pH acide, notamment inférieur à 2 et plus particulièrement inférieur à 1,2 ainsi qu'aux attaques des enzymes gastriques.

Bien entendu, les constituants du véhicule doivent posséder comme caractéristiques de pouvoir être modifiés ou dégradés de manière spécifique dans la lumière de l'intestin, c'est-à-dire à un pH supérieur à environ 7,8 et en présence des enzymes entériques, afin de libérer le vecteur (nanoparticule) dans la lumière de l'intestin.

Un vecteur complexe particulier selon la présente invention est un vecteur complexe dans lequel le véhicule est sous forme d'une matrice sphérique.

De préférence, le vecteur complexe est sous forme de sphères d'environ 1,5 mm de diamètre.

Dans le cadre de la présente invention, le véhicule tel que défini ci-dessus peut être sous forme d'une capsule sphérique.

Selon un mode de réalisation particulièrement préféré, lorsque le véhicule est sous forme d'une matrice sphérique ou d'une capsule sphérique, celui-ci est à base d'alginate, notamment sous la forme d'une sphère ou d'une capsule d'alginate.

Dans le cadre de la présente invention, le véhicule peut être sous forme d'une matrice sphérique obtenue par extrusion d'une solution d'alginate de sodium dans un bain de CaCl₂.

Par ailleurs, tous les polymères gélifiants biocompatibles, bioassimilables et/ou métabolisables peuvent être employés pour former les matrices sphériques ou les capsules sphériques.

La présente invention concerne également un vecteur complexe tel que défini ci-dessus, dans lequel le véhicule contient un dispersant lipophile.

En outre, le véhicule gastro-résistant peut éventuellement renfermer un milieu lipophile, dans lequel le ou les vecteurs (nanoparticules) définis précédemment sont présents. Ce milieu lipophile peut être sous forme solide, liquide ou encore sous forme de gel. Le milieu lipophile peut être constitué de tout composé lipophile connu en soi et non toxique du point de vue pharmacologique. Le composé lipophile envisagé peut par exemple être choisi parmi les huiles organiques ou minérales, végétales ou animales, par exemple, l'huile d'olive, l'huile de foie de morue, huiles silicone, et autres, ainsi que leurs mélanges.

De façon préférée, le dispersant lipophile susmentionné est choisi dans le groupe constitué des huiles organiques ou minérales, végétales ou animales, et leurs mélanges.

Avantageusement, le dispersant lipophile susmentionné est un mélange d'esters d'acides gras, notamment choisis dans le groupe constitué des acides caproïque, caprylique, caprique, laurique, myristique, linoléique et succinique.

La présente invention concerne également une composition pharmaceutique comprenant au moins une nanoparticule telle que définie ci-dessus ou au moins un vecteur complexe tel que défini ci-dessus, en association avec un véhicule pharmaceutiquement acceptable.

La présente invention concerne également l'utilisation d'au moins une nanoparticule telle que définie ci-dessus ou au moins un vecteur tel que défini ci-dessus, à titre de médicament.

La présente invention concerne également l'utilisation d'au moins une nanoparticule telle que définie ci-dessus ou au moins un vecteur complexe tel que défini ci-dessus, pour la préparation d'un médicament destiné au traitement de maladies auto-immunes, et notamment du lupus érythémateux disséminé.

La présente invention concerne une nanoparticule telle que définie ci-dessus pour son utilisation pour le traitement des maladies auto-immunes, et notamment du lupus érythémateux disséminé.

La présente invention concerne également un vecteur complexe tel que défini ci-dessus pour son utilisation pour le traitement des maladies auto-immunes, et notamment du lupus érythémateux disséminé.

### PARTIE EXPÉRIMENTALE

### PRÉPARATION DES NANOPARTICULES HAP140

Les nanoparticules HAP140 sont des nanoparticules obtenues par coacervation complexe entre l'acide hyaluronique (HA) et le peptide P140.

L'acide hyaluronique (5,2 mg ; CERMAV Grenoble, France - masse molaire 27 600 Da) est dissous dans 25 mL d'eau bidistillée et laissé sous agitation pendant 24 heures afin d'assurer une bonne dissolution du polymère HA. Le peptide P140 (200 µg) est pesé sur une microbalance puis dissous dans 250 µL d'eau bidistillée (pH 5,5). Il est ensuite ajouté goutte à goutte à l'aide d'une micropipette à la solution d'acide hyaluronique (1,05 mL ; pH 6,5) dans un tube à essais muni d'un petit barreau aimanté tournant à 250 tr.min⁻¹. Le tube à hémolyse contenant le peptide est lavé avec 50 µL d'eau bidistillée qui sont ensuite ajoutés à la suspension.

L'addition du peptide P140 à l'acide hyaluronique s'accompagne de la formation progressive d'un trouble dans la solution. Après 15 minutes d'agitation, la suspension est transférée dans un micro-tube de 1,5 mL et conservée à 4°C. Aucun agrégat n'étant formé, aucune étape de filtration n'est nécessaire.

Les nanoparticules ainsi obtenues ont les propriétés suivantes (distribution en taille et indice de polydispersité) :

| HA | taille (nm) | % en nombre | indice de polydispersité |
|---|---|---|---|
| 27 600 Da | 220 | 100 | 0,07 |

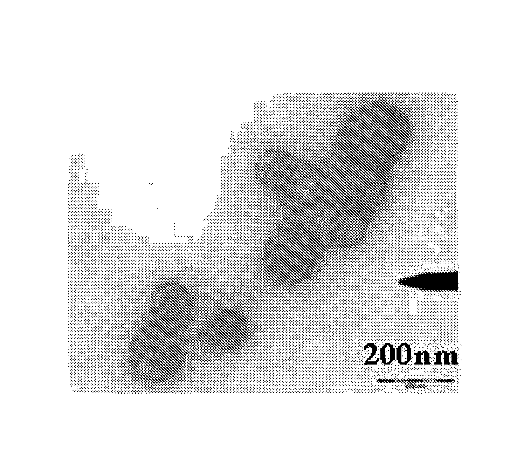

Ces nanoparticules sont parfaitement sphériques (Figure 1 - cliché des nanoparticules obtenues HAP140 par microscopie électronique à transmission) et très monodisperses.

De préférence, ces nanoparticules sont isolées 24 heures après leur synthèse afin d'assurer une bonne efficacité d'encapsulation puis sont conservées pendant une semaine à 4°C dans de l'eau bidistillée afin d'atteindre leur équilibre thermodynamique et d'être stables en milieu salin et en milieu intestinal simulé.

### ADMINISTRATION DE NANOPARTICULES HAP140 CHEZ DES SOURIS BALB/c

La synthèse des nanoparticules HAP140 étant mise au point, il a été vérifié que le peptide conserve son intégrité lorsqu'il est condensé par l'acide hyaluronique sous forme de nanoparticules.

Il a été montré que le peptide P140 est immunogène lorsqu'il est administré en présence d'adjuvant chez les souris BALB/c. Afin de vérifier que le peptide P140 est toujours reconnu par les cellules du système immunitaire, notamment les cellules T, lorsqu'il est sous forme de particules, il a été choisi d'administrer les nanoparticules HAP140 en présence d'adjuvant à des souris BALB/c et de comparer la réponse immunitaire induite par l'administration en présence d'adjuvant de nanoparticules HAP140 à celle obtenue par une administration de peptide P140 libre.

### 1. Développement de la réponse immunitaire

Lorsqu'un antigène pénètre l'organisme, il est détecté puis internalisé par les cellules dendritiques et les macrophages présents sur le site d'infection (ou d'administration). Ces cellules deviennent matures et rejoignent les vaisseaux du système lymphatique où circule la lymphe qui transporte les déchets et les corps étrangers. Elles sont acheminées vers les ganglions lymphatiques, des organes lymphoïdes secondaires colonisés par les lymphocytes B et T. C'est au niveau de ces ganglions drainant le site d'infection (ou d'immunisation) que la réponse immune s'élabore et que les cellules T sont activées suite à la présentation de l'antigène par les cellules dendritiques ou autres cellules présentatrices d'antigènes.

La plupart des protéines ou des peptides comme le P140 sont peu immunogènes lorsqu'ils sont administrés seuls en absence de composants représentant des signaux de danger. Afin d'obtenir une forte réponse immunitaire dirigée contre un antigène protéique, il est donc nécessaire de l'administrer sous forme de mélange avec un adjuvant. La plupart des adjuvants contiennent des bactéries tuées ou des composants bactériens qui stimulent les macrophages et favorisent la réponse immune. L'adjuvant utilisé ici est l'adjuvant complet de Freund (CFA). Il s'agit d'une huile contenant des mycobactéries mortes qui non seulement facilitent la présentation de l'antigène par les macrophages ou les cellules dendritiques mais induisent également la production de cytokines inflammatoires et de fortes réactions inflammatoires locales.

### 2. Administration des nanoparticules HAP140 par voie sous-cutanée

L'administration sous-cutanée (s.c.) de peptide P140 en présence d'adjuvant à des souris BALB/c conduit à l'activation des cellules T auxiliaires Th [Monneaux, F., Lozano, J.M., Patarroyo, M.E., Briand, J.P., and Muller, S. (2003) T cell recognition and therapeutic effect of a phosphorylated synthetic peptide of the 70K snRNP protein administered in MRL/lpr mice. Eur. J. Immunol. 33, 287-296]. Cette activation est mise en évidence par l'analyse de la prolifération et de la production de cytokines des cellules T présentes dans les ganglions drainant le site d'administration, stimulées *ex vivo* avec le peptide P140.

### a) Protocole

Des souris BALB/c femelles âgées de 7 semaines (4 groupes de deux souris) sont immunisées au niveau des flancs avec différentes formulations par voie s.c. Le premier groupe a reçu le peptide P140 en présence de CFA (groupe contrôle), le deuxième groupe a reçu les nanoparticules HAP140 en présence de CFA, le troisième groupe a reçu les nanoparticules HAP140 en milieu salin (afin de vérifier que les particules HAP140 ne présentent pas de caractère adjuvant à elles-seules), et le quatrième a reçu les nanoparticules « blanches » HACS en présence de CFA (afin de vérifier la spécificité de la réponse induite vis-à-vis du peptide P140). Les souris ayant reçu du peptide sous sa forme libre ou sous forme de nanoparticules, ont toutes reçu la même quantité de peptide, à savoir 100µg par souris.

Une semaine après l'immunisation, les souris sont sacrifiées et les ganglions lymphatiques du flanc et de la patte avant sont prélevés. Les cellules issues de ces ganglions sont mises en culture *ex vivo* à raison de 5.10⁵ cellules par puits en présence de différentes concentrations de peptide (0, 6, 20 et 60µM) afin d'étudier leur prolifération ainsi que la sécrétion de cytokines.

Les cytokines sont des protéines de faible masse molaire produites par de nombreuses populations cellulaires, dont les lymphocytes. Elles peuvent être décrites comme les « hormones » du système immunitaire puisqu'elles interviennent dans les relations entre les lymphocytes, les macrophages et autres cellules. Leurs fonctions sont très diverses : elles peuvent influencer la survie, la prolifération, la différenciation ou encore la migration des cellules.

Les cellules Th sont classées en sous-populations, Th1 ou Th2, suivant les cytokines qu'elles produisent et la fonction qu'elles exercent. Les cellules de type Th-1 produisent notamment de l'interleukine-2 (IL-2) et de l'interféron-γ (IFN-γ) tandis que les cellules de type TH-2 sécrètent essentiellement de l'IL-4, de l'IL-5, de l'IL-6, de l'IL10 et de l'IL-13. Les conditions d'immunisation utilisées ici, c'est-à-dire l'utilisation de CFA, favorisent le développement d'une réponse de type Th1. On a donc étudié uniquement la sécrétion d'IL-2 et d' IFN-γ.

Si l'immunisation a été efficace, les cellules Th des ganglions drainant le site de l'immunisation seront activées et vont, en présence de peptide P140, proliférer et sécréter des cytokines.

### b) Résultats

Deux formulations, le peptide en présence d'adjuvant et les particules HAP140 en présence d'adjuvant, ont donné des indices de stimulation positifs et dose-dépendants, ce qui indique une réponse proliférative spécifique du peptide P140. Les indices de stimulation associés aux particules administrées en solution saline restent très faibles en comparaison, tandis que ceux associés aux particules « blanches » sont négatifs.

Les résultats de sécrétion des cytokines confirment ceux de la prolifération : deux réponses dose-dépendantes ont été obtenues dans le cas de l'administration du peptide et des nanoparticules HAP140 tous deux injectés en présence de CFA. Les particules administrées seules, mènent uniquement à la production d'une faible quantité d'IL-2 qui n'est pas dose-dépendante et probablement non significative, et à aucune production d'IFN-γ. L'administration de particules « blanches » n'induit la sécrétion d'aucune cytokine.

Le peptide est donc libéré des nanoparticules HAP140 sous sa forme native puisque l'administration s.c. de celle-ci en présence de CFA engendre la même réponse spécifique que l'administration du peptide libre en présence de CFA. Les indices de stimulation obtenus ainsi que les concentrations en cytokines sécrétées sont similaires pour ces deux informations. Il semble donc que l'intégralité du peptide condensé dans les particules soit préservée après une administration s.c. aux souris.

De plus, on montre que l'acide hyaluronique ne présente aucun caractère adjuvant, la co-administration de CFA étant nécessaire à l'obtention de réponses dose-dépendantes. Ceci est très important car dans le cadre de la mise en place de protocoles thérapeutiques chez la souris lupique, tout effet adjuvant est à proscrire puisqu'il aurait pour conséquence potentielle d'accélérer le développement de la maladie [Monneaux, et al. (2003) Eur. J. Immunol. 33, 287-296].

Enfin, l'administration de particules « blanches » HACS ne génère aucune réponse immunitaire démontrant ainsi que la réponse mesurée est bien spécifique du peptide P140 et non pas des particules seules.

### 3. Administration des nanoparticules HAP140 par voie intra-duodénale

D'après les résultats obtenus dans le paragraphe précédent, la formation des nanoparticules HAP140 se fait dans des conditions non dégradantes pour le peptide et celui-ci est libéré sous sa forme native dans l'organisme après une administration s.c. Cette voie d'administration est cependant éloignée de la voie d'administration envisagée, à savoir la voie orale.

Dans le vecteur pharmaceutique complexe décrit précédemment, les nanoparticules sont protégées par un véhicule acido-résistant dans l'estomac, puis libérées dans l'intestin afin de traverser l'épithélium intestinal. Afin de mimer l'utilisation de ce vecteur, les nanoparticules sont administrées dans le segment initial de l'intestin grêle, le duodénum. Cette voie d'administration permet de vérifier que les nanoparticules sont stables en milieu intestinal, que le peptide est protégé des attaques enzymatiques et qu'une fraction au moins de celui-ci traverse intacte la barrière intestinale.

### a) Protocole

Des souris BALB/c femelles âgées de 7 semaines sont immunisées avec différentes formulations par différentes voies d'administration. Le premier groupe a reçu le peptide P140 en présence de CFA par voie s.c., le deuxième groupe a reçu les nanoparticules HAP140 en présence de CFA par voie intra-duodénale et le troisième groupe a reçu les nanoparticules « blanches » HACS en présence de CFA par voie intra-duodénale. Les souris ayant reçu du peptide, soit sous sa forme libre, soit sous forme de nanoparticules, ont toutes reçu la même quantité de peptide, à savoir 100µg par souris, quelle que soit la voie d'administration utilisée.

L'immunisation par voie intra-duodénale se fait sous anesthésie. Une légère incision est alors pratiquée au niveau de l'abdomen afin de sortir le segment supérieur de l'intestin de la cavité abdominale et de réaliser l'administration dans le duodénum. L'incision est ensuite recousue et l'animal est gardé sous une lampe chauffante jusqu'à son réveil.

Une semaine après l'immunisation, les souris sont sacrifiées et les ganglions drainant le site d'administration sont prélevés. Dans le cas de l'administration intra-duodénale, il s'agit des ganglions mésentériques qui forment une chaîne le long de l'intestin et drainent la muqueuse intestinale. Les cellules issues de ces ganglions sont mises en culture en présence de différentes concentrations de peptide P140 suivant le protocole décrit dans le paragraphe précédent.

### b) Résultats

Les résultats obtenus par voie intra-duodénale ont été superposés à ceux obtenus précédemment par voie s.c. pour permettre une comparaison. Il est à noter que les ganglions mésentériques des souris ayant reçu les particules HAP140 en présence de CFA par voie intra-duodénale sont hypertrophiés, signe qu'ils ont été le site d'une réponse immunitaire.

La prolifération des cellules issues des souris immunisées avec les particules HAP140 par voie intra-duodénale en présence d'adjuvant est bien dose-dépendante et les indices de stimulation sont du même ordre de grandeur que ceux obtenus par administration s.c. du peptide P140 libre en présence d'adjuvant. Les indices de stimulation associés aux particules « blanches » HACS administrées par voie intra-duodénale ne sont pas significatifs, quelle que soit la concentration en peptide, signe que les cellules ganglionnaires n'ont pas proliféré.

L'administration intra-duodénale de particules HAP140 en présence d'adjuvant entraîne une sécrétion de cytokines de type Th1 plus faible que dans le cas d'une administration sous-cutanée. Ceci est particulièrement évident pour l'IFN-γ. Cependant, les sécrétions d'IL-2 et d'IFN-γ restent dose-dépendantes et les indices de prolifération cellulaire sont du même ordre de grandeur que ceux obtenus par voie s.c. L'administration de nanoparticules HAP140 par voie intra-duodénale permet donc d'activer les cellules Th des ganglions mésentériques.

Ainsi, le peptide est capable d'activer efficacement les cellules Th, signe qu'il est sous forme native. Les particules sont donc être suffisamment stables en milieu intestinal *in vivo* pour protéger le peptide des attaques enzymatiques.

### PROTOCOLE THÉRAPEUTIQUE CHEZ LA SOURIS LUPIQUE MRL/Ipr PAR ADMINISTRATION INTRA-DUODÉNALE DE NANOPARTICULES HAP140

Afin d'étudier l'efficacité thérapeutique de l'administration intra-duodénale des nanoparticules HAP140, un protocole thérapeutique a été entrepris chez la souris lupique.

Afin d'étudier le potentiel thérapeutique des nanoparticules HAP140, des souris prélupiques ont reçu trois administrations de ces particules par voie intra-duodénale en l'absence d'adjuvant, espacées chacune de deux semaines. Pour permettre une comparaison de l'évolution de la maladie et étudier l'efficacité de nos nanoparticules, un groupe de souris a reçu des nanoparticules HACS sans peptide dites « blanches ».

### Protocole

Des souris pré-lupiques MRL/Ipr femelles âgées de 5 semaines au début des expériences ont été séparées en deux groupes. Le premier groupe, constitué de 8 souris, a reçu 100µg de P140 sous forme de nanoparticules HAP140 par voie intra-duodénale et le second, groupe contrôle constitué de 5 souris, a reçu 100µg de chitosane sous forme de nanoparticules HACS également par voie intra-duodénale. La première administration se fait à l'âge de 5 semaines et elle est suivie de deux autres administrations espacées de deux semaines (soit à l'âge de 7 et 9 semaines). Les symptômes de la maladie lupique, comme l'apparition notamment d'une protéinurie (présence de protéines dans les urines mesurée sur bandelette urinaire), ont été suivis régulièrement pendant plus de 45 semaines et un profil de mortalité a été établi.

### Résultats

L'administration intra-duodénale de nanoparticules HAP140 retarde l'apparition de la protéinurie chez les souris lupiques MRU/Ipr. En effet, ce symptôme n'apparaît qu'à l'âge de 15 semaines chez les souris traitées et 12 semaines chez le groupe de souris contrôle non traitées. Leur protéinurie reste également toujours moins fréquente que celle développée par les souris contrôle.

L'administration des nanoparticules HAP140 semble aussi avoir un effet bénéfique sur la mortalité des animaux traités. Tout d'abord, l'administration retarde significativement l'amorçage de la courbe de mortalité. En effet, si dans le groupe contrôle, les premières souris meurent à 15 semaines, ce n'est qu'à 22 semaines que la première souris est morte dans le groupe ayant reçu les nanoparticules HAP140. Cet écart est particulièrement intéressant si l'on considère que d'après les données de la littérature, la durée de vie d'une souris lupique MRL/Ipr n'excède pas les 35 semaines.

Ces données sont par ailleurs confirmées dans notre étude, puisqu'à l'âge de 37 semaines, toutes les souris du groupe contrôle sont mortes. De façon intéressante, 50% des souris ayant reçu les nanoparticules sont encore vivantes à cet âge, elles le sont toujours à 45 semaines soit une demi-vie des souris non traitées de 25 semaines, à comparer à une demi-vie des souris traitées de 45 semaines.

L'administration de nanoparticules HAP140 par voie intra-duodénale chez des souris pré-lupiques permet donc d'allonger de façon significative leur durée de vie.

Ainsi, l'administration intra-duodénale de nanoparticules HAP140 permet non seulement de retarder l'apparition de signes cliniques de la maladie comme la protéinurie mais elle permet également d'allonger la durée de vie des animaux de manière très significative.

Une partie au moins des particules ou de la dose de peptide est donc capable de traverser la barrière intestinale et d'atteindre la circulation systémique et/ou les cellules cibles.

### ETUDE DU FRANCHISSEMENT DE LA BARRIÈRE INTESTINALE

Afin d'étudier le passage des nanoparticules à travers la paroi intestinale, un modèle *in vitro* utilisant une lignée de cellules humaines cancéreuses de colon (Caco-2) a été développé. Ces cellules sont capables de se différencier en entérocytes durant leur culture, mimant ainsi la couche de cellules constituant la paroi intestinale.

Les observations au microscope électronique à transmission ont pu mettre en évidence la différenciation de ces cellules après 13 jours de culture (présence de villosités et de desmosomes). Ces cellules contiennent beaucoup d'organites d'une taille proche de celle des nanoparticules synthétisées ce qui rend leur identification difficile.

Des observations par microscopie confocale ont donc été effectuées afin d'observer le passage des particules à travers ce modèle d'épithélium. Pour cela, le chitosane a été couplé à l'isothiocyanate de fluorescéine (FITC) et des nanoparticules HACS_{FITC} ont été synthétisées. Ces particules possèdent des caractéristiques (taille, état de surface) proches de celles des particules HAP140 et peuvent être obtenues en quantité suffisante pour les études envisagées. Les observations en microscopie confocale ont démontré la présence de nanoparticules non seulement en surface des cellules mais également dans leur cytoplasme. Il a été montré que le passage des particules dans les cellules se fait par un mécanisme probablement actif puisque celui-ci a lieu à 37°C mais pas à 4°C. Des études par cytométrie en flux ont montré par ailleurs que l'absorption des particules se fait non seulement de façon dose-dépendante mais augmente également avec la durée d'incubation. La présence des nanoparticules dans le cytoplasme des cellules Caco-2 a été confirmée par microscopie électronique à transmission grâce à l'utilisation d'un anticorps anti-FITC couplé à des billes d'or.

Ce modèle cellulaire présente cependant des différences vis-à-vis d'un épithélium intestinal, comme l'absence de mucus ou de cellules M. Des nanoparticules fluorescentes ont donc été administrées par voie intraduodénale à des souris lupiques et des coupes d'intestin ont été réalisées afin d'observer le passage de ces nanoparticules. Une forte intensité de fluorescence est retrouvée au niveau de la lumière intestinale et du mucus, connu pour piéger les particules. Des particules fluorescentes sont également détectées à l'intérieur des villosités et ce jusqu'à la membrane basale de l'intestin. Ainsi, si la majorité des particules reste au niveau du mucus, une quantité non négligeable d'entre elles sont capables de traverser l'épithélium intestinal.

### SYNTHÈSE D'UN VÉHICULE ACIDO-RÉSISTANT EN ALGINATE CONTENANT UNE HUILE PHARMACEUTIQUE

### 1. Protocole de synthèse du véhicule

### Principe de la synthèse

Le protocole utilisé pour l'obtention d'un tel véhicule est le suivant : du carbonate de calcium finement broyé est dispersé dans une huile pharmaceutique de type Miglyol^{®}. Cette suspension est ajoutée goutte à goutte à un débit de 20mL/h par l'intermédiaire d'une seringue munie d'une aiguille en inox (diamètre de l'aiguille 0,9 mm) dans un bain d'alginate de sodium contenant de l'acide acétique glacial. En contact avec cette solution acide, le carbonate de calcium va libérer des ions calcium à l'interface huile/eau qui vont alors se complexer avec l'alginate et former une membrane autour de chaque goutte d'huile. Les capsules ainsi obtenues auront donc une taille de quelques millimètres, voisine de celle de la goutte d'huile formée en sortie de l'aiguille.

100 mL d'une solution d'alginate de très faible viscosité à 0,5% (w/v) contenant 1 % (v/v) d'acide acétique glacial sont versés dans un petit réacteur de 250mL muni d'un agitateur mécanique en forme d'ancre. Le carbonate de calcium (500 mg) est finement dispersé dans 5 mL de Miglyol^{®} 829 (densité 1 à 1,02). Cette dispersion est ensuite ajoutée goutte à goutte par l'intermédiaire d'une seringue munie d'une aiguille en inox (diamètre de l'aiguille 0,9 mm) dans la solution acide d'alginate à un débit constant de 20 mL/h. Une fois l'ajout terminé, le système est laissé sous agitation à 150 tr/min pendant une heure afin de laisser le temps aux ions calcium de migrer vers la solution aqueuse et de complexer l'alginate en surface des gouttes d'huile. Les véhicules sont ensuite dilués dans un grand volume d'eau pour faciliter leur filtration sur système de filtration, par exemple Millipore (3 µm). Afin de renforcer la réticulation de l'alginate, ils sont ensuite immergés dans une solution de chlorure de calcium pendant 30 min puis conservés à 4°C dans de l'eau. Des véhicules sphériques d'un diamètre compris entre 1,5 mm et 2 mm et d'une épaisseur de membrane proche de 500 µm sont ainsi obtenus.

### 2. Stabilité du véhicule en milieux physiologigues reconstitués

La stabilité de ces véhicules a été évaluée en milieux gastrique et intestinal simulés. Nous avons tout particulièrement étudié l'influence de la durée de synthèse des véhicules (durée de mise en contact des gouttes d'huile contenant le carbonate de calcium avec la solution acide d'alginate) sur leur stabilité.

L'immersion de ces véhicules en milieu gastrique simulé conduit à une diminution de l'épaisseur de leur membrane en alginate visible à l'oeil nu. Ce phénomène est similaire à celui observé pour le premier type de véhicule développé dans ce chapitre : à pH acide, l'alginate précipite en acide alginique, ce qui s'accompagne d'une rétractation du gel. La membrane des véhicules mise en contact avec l'alginate pendant au moins 30 min reste intact après 4 heures d'immersion en milieu gastrique simulé. Ce n'est pas le cas pour les véhicules dont la durée de synthèse est inférieure à 15 minutes : la mince capsule d'alginate ainsi obtenue se fend et libère son contenu, à savoir l'huile, dans ce milieu acide. Les véhicules doivent donc être conservés au moins 30 minutes dans le bain d'alginate afin d'être stables durant 4 heures en milieu gastrique simulé.

Lorsque les véhicules sont transférés en milieu intestinal simulé, on observe une rupture très rapide des membranes des véhicules, amincies par leur séjour en milieu acide. En moins de 30 minutes, tous les véhicules sont dégradés et l'intégralité de l'huile est libérée dans le milieu. La dégradation est d'autant plus rapide que les véhicules sont restés peu de temps en contact avec la solution d'alginate au cours de leur synthèse. Ainsi, des véhicules immergés pendant 1 heure dans le bain d'alginate se dégradent en 30 minutes en milieu intestinal simulé tandis que 5 minutes sont suffisantes pour dégrader l'intégralité des véhicules immergés seulement 30 minutes dans ce même bain d'alginate.

**Tableau 1 :**

| Influence de la durée de réticulation de l'alginate de calcium sur la stabilité des véhicules obtenus en milieu gastrique simulé puis en milieu intestinal simulé | | |
|---|---|---|
| **Durée de réticulation de l'alginate** | **Stabilité en milieu gastrique simulé (4 h)** | **Stabilité en milieu intestinal simulé** |
| 15 min | Non | X |
| 30 min | Oui | 5 min |
| 45 min | Oui | 15 min |
| 1 h | Oui | 30 min |

Il est donc possible de jouer sur le temps d'immersion des véhicules dans le bain d'alginate lors de leur synthèse pour accélérer ou ralentir le phénomène de dégradation observé en milieu intestinal simulé.

La dégradation des véhicules en milieu intestinal simulé doit être suffisamment rapide pour avoir lieu dans le segment supérieur de l'intestin : si la dégradation du véhicule n'a pas lieu suffisamment tôt, les nanoparticules y resteront piégées durant une partie de leur séjour dans l'intestin et ne pourront entrer en contact avec l'épithélium intestinal. Une durée d'incubation de 30 à 45 min dans le bain d'alginate semble être optimale puisqu'elle permet d'obtenir des véhicules non seulement stables 4 heures en milieu gastrique mais qui semblent également se dégrader rapidement (5 à 15 min) en milieu intestinal simulé.

Ce nouveau véhicule remplit donc *in vitro* ses deux fonctions principales : résister à un pH acide pendant 4 heures et ainsi protéger les nanoparticules pendant leur passage dans l'estomac, et se dégrader rapidement en milieu intestinal afin de les libérer.

### 3.- Miniaturisation du véhicule - étude préliminaire

Dans le but de l'administration du vecteur complexe par voie orale à des souris, il est nécessaire de miniaturiser le véhicule.

### a) Principe de la synthèse

L'obtention de petites capsules d'alginate à coeur huileux est basée sur une simple modification du protocole utilisé précédemment : une émulsion huile dans eau est obtenue par homogénéisation d'une suspension de carbonate de calcium dans du Miglyol^{®} 829 avec une solution aqueuse d'alginate. L'addition d'acide acétique glacial dans cette émulsion permet d'initier la migration du carbonate de calcium vers l'alginate et la libération de cations Ca²⁺, menant alors à la formation de membranes d'alginate réticulé autour de chacune des gouttelettes d'huile.

### b) Protocole de synthèse

Le CaCO₃ (100mg) est dispersé dans 1 mL d'huile pharmaceutique Miglyol^{®} 829, puis cette dispersion est homogénéisée dans une solution d'alginate de très faible viscosité à 0,5% (w/v). L'émulsion est obtenue à l'aide d'un homogénéisateur (Ultra-Turrax^{®} T25 basik IKA^{®} Werke) utilisé à une vitesse de 600 tr/min pendant 5 minutes. L'acide acétique glacial (500 µL) est alors ajouté goutte à goutte afin d'initier la libération des cations calcium et la formation de la membrane en alginate autour des gouttelettes.

Ces conditions mènent à la formation de capsules sphériques d'alginate à coeur huileux d'une taille de quelques dizaines de µm.

### PRÉPARATION D'UN VECTEUR SELON L'INVENTION

Le protocole de synthèse d'un vecteur complexe est identique à celui des véhicules en remplaçant l'huile pharmaceutique Miglyol^{®} 829 par une dispersion de nanoparticules contenant le peptide P140 (ou analogue) dans l'huile pharmaceutique Miglyol^{®} 829, le restant du protocole demeurant inchangé.

## Revendications

1. Nanoparticule comprenant une matrice constituée d'au moins un polysaccharide et du peptide P140 de séquence SEQ ID NO : 1 ou d'un de ses analogues, ledit polysaccharide étant de préférence un polysaccharide porteur de groupement(s) fonctionnel(s) négativement chargé(s).

2. Nanoparticule selon la revendication 1, **caractérisée en ce que** le polysaccharide est l'acide hyaluronique.

3. Nanoparticule selon la revendication 1 ou 2, dont la taille est comprise d'environ 1 nm à environ 10 µm.

4. Nanoparticule selon l'une quelconque des revendications 1 à 3, **caractérisée en ce que** le rapport massique entre le polysaccharide et le peptide est compris d'environ 0,1 à environ 10, et notamment compris d'environ 0,5 à environ 5, et de préférence égal à environ 1.

5. Nanoparticule selon l'une quelconque des revendications 1 à 4, **caractérisée en ce que** la masse molaire du polysaccharide est comprise d'environ 1 000 Da à environ 1 500 000 Da, et de préférence d'environ 15 000 Da à environ 50 000 Da, et notamment d'environ 20 000 Da à environ 30 000 Da.

6. Vecteur complexe pour administration par voie orale constitué d'un véhicule qui comprend au moins une nanoparticule selon l'une quelconque des revendications 1 à 5.

7. Vecteur complexe selon la revendication 6, **caractérisé en ce qu'**il comprend un véhicule destiné à une protection gastrique.

8. Vecteur complexe selon la revendication 7, **caractérisé en ce que** le véhicule est sous forme d'une matrice sphérique ou d'une capsule sphérique.

9. Vecteur complexe selon la revendication 8, **caractérisé en ce que** le véhicule est à base d'alginate notamment sous la forme d'une sphère ou d'une capsule d'alginate.

10. Vecteur complexe selon la revendication 7, **caractérisé en ce que** le véhicule contient un dispersant lipophile, notamment choisi dans le groupe constitué des huiles organiques ou minérales, végétales ou animales, et leurs mélanges, et est de préférence un mélange d'esters d'acides gras, notamment choisis dans le groupe constitué des acides caproïque, caprylique, caprique, laurique, myristique, linoléique et succinique.

11. Composition pharmaceutique comprenant au moins une nanoparticule selon l'une quelconque des revendications 1 à 5 ou au moins un vecteur complexe selon l'une quelconque des revendications 6 à 10, en association avec un véhicule pharmaceutiquement acceptable.

12. Médicament comprenant au moins une nanoparticule selon l'une quelconque des revendications 1 à 5 ou au moins un vecteur complexe selon l'une quelconque des revendications 6 à 10.

13. Nanoparticule selon l'une quelconque des revendications 1 à 5, pour son utilisation pour le traitement de maladies auto-immunes, et notamment du lupus érythémateux disséminé.

14. Vecteur complexe selon l'une quelconque des revendications 6 à 10, pour son utilisation pour le traitement de maladies auto-immunes, et notamment du lupus érythémateux disséminé.

## Claims

1. Nanoparticle comprising a matrix consisting of at least one polysaccharide and of the peptide P140 having the sequence SEQ ID NO: 1 or one of the analogs thereof, said polysaccharide preferably being a polysaccharide carrying one or more negatively charged functional groups.

2. Nanoparticule according to claim 1, **characterized in that** the polysaccharide is hyaluronic acid.

3. Nanoparticule according to claim 1 or 2, the size of which is from around 1 nm to around 10 µm.

4. Nanoparticle according to any one of claims 1 to 3, **characterized in that** the weight ratio between the polysaccharide and the peptide is from around 0.1 to around 10, and in particular from around 0.5 to around 5, and preferably equal to around 1.

5. Nanoparticle according to any one of claims 1 to 4, **characterized in that** the polar mass of the polysaccharide is from around 1000 Da to around 1 500 000 Da, and preferably from around 15 000 Da to around 50 000 Da, and in particular from around 20 000 Da to around 30 000 Da.

6. Complex vector for oral administration, consisting of a carrier which comprises at least one nanoparticle according to any one of claims 1 to 5.

7. Complex vector according to claim 6, **characterized in that** it comprises a carrier intended for gastric protection.

8. Complex vector according to claim 7, **characterized in that** the carrier is in the form of a spherical matrix or a spherical capsule.

9. Complex vector according to claim 8, **characterized in that** the carrier is alginate-based, in particular in the form of an alginate sphere or capsule.

10. Complex vector according to claim 7, **characterized in that** the carrier contains a lipophilic dispersant, in particular selected from the group consisting of organic or mineral, plant or animal oils, and mixtures thereof, and is preferably a mixture of fatty acid esters, in particular selected from the group consisting of caproic, caprylic, capric, lauric, myristic, linoleic and succinic acids.

11. Pharmaceutical composition comprising at least one nanoparticle according to any one of claims 1 to 5 or at least one complex vector according to any one of claims 6 to 10, in combination with a pharmaceutically acceptable carrier.

12. Drug comprising at least one nanoparticle according to any one of claims 1 to 5 or at least one complex vector according to any one of claims 6 to 10,

13. Nanoparticle according to any one of claims 1 to 5 for its use for the treatment of autoimmune diseases, in particular systemic lupus erythematosus.

14. Complex vector according to any one of claims 6 to 10 for its use for the treatment of autoimmune diseases, in particular systemic lupus erythematosus.

## Patentansprüche

1. Nanopartikel, das eine Matrix aufweist, die aus mindestens einem Polysaccharid und einem Peptid P140 der Sequenz SEQ ID NO:1 oder einem seiner Analoge gebildet ist, wobei das Polysaccharid vorzugsweise ein Polysaccharid ist, welches Träger von einer oder mehreren negativ geladenen funktionellen Gruppe(n) ist.

2. Nanopartikel nach Anspruch 1, **dadurch gekennzeichnet, dass** das Polysaccharid die Hyaluronsäure ist.

3. Nanopartikel nach Anspruch 1 oder 2, dessen Größe ungefähr zwischen 1 nm bis ungefähr 10 µm liegt.

4. Nanopartikel nach einem beliebigen der Ansprüche 1 bis 3, **dadurch gekennzeichnet, dass** das Massenverhältnis zwischen dem Polysaccharid und dem Peptid zwischen ungefähr 0,1 bis ungefähr 10 liegt und insbesondere zwischen ungefähr 0,5 bis ungefähr 5 liegt und vorzugsweise gleich ungefähr 1 ist.

5. Nanopartikel nach einem beliebigen der Anspruche 1 bis 4, **dadurch gekennzeichnet, dass** die Molmasse des Polysaccharids zwischen ungefähr 1 000 Da und 1 500 000 Da und bevorzugt zwischen ungefähr 15 000 Da und 50 000 und insbesondere zwischen ungefähr 20 000 Da und 30 000 Da liegt.

6. Komplexer Vektor für die orale Darreichung bestehend aus einer Trägersubstanz, die mindestens ein Nanopartikel nach einem beliebigen der Ansprüche 1 bis 5 umfasst.

7. Komplexer Vektor nach Anspruch 6, **dadurch gekennzeichnet dass** er eine für einen Magenschutz vorgesehen Trägersubstanz umfasst.

8. Komplexer Vektor nach Anspruch 7, **dadurch gekennzeichnet dass** die Trägersubstanz in Form einer kugelförmigen Matrix oder einer kugelförmigen Kapsel ausgebildet ist.

9. Komplexer Vektor nach Anspruch 8, **dadurch gekennzeichnet dass** die Trägersubstanz auf Alginatbasis ist, insbesondere in Form einer Alginatkugel oder einer Alginatkapsel ausgestaltet ist.

10. Komplexer Vektor nach Anspruch 7, **dadurch gekennzeichnet dass** die Trägersubstanz ein lipophiles Dispergiermittel enthält, insbesondere ausgewählt aus der Gruppe, die von organischen oder mineralischen, pflanzlichen oder tierischen Ölen und ihren Mischungen gebildet ist, und vorzugsweise eine Mischung aus Fettsäureestern ist, insbesondere ausgewählt aus der Gruppe, die von Capronsäuren, Caprylsäuren, Caprinsäuren, Laurinsäuren, Myristinsäuren, Linolsäuren und Bernsteinsäuren gebildet ist.

11. Pharmazeutische Zusammensetzung, mindestens ein Nanopartikel nach einem beliebigen der Ansprüche 1 bis 5 oder mindestens einen komplexen Vektor nach einem beliebigen der Ansprüche 6 bis 10 in Zuordnung mit einer annehmbaren, pharmazeutischen Trägersubstanz umfassend.

12. Medikament, mindestens ein Nanopartikel nach einem beliebigen der Ansprüche 1 bis 5 oder mindestens einen komplexen Vektor nach einem beliebigen der Ansprüche 6 bis 10 umfassend.

13. Nanopartikel nach einem beliebigen der Ansprüche 1 bis 5 für seine Anwendung zur Behandlung von Autoimmunkrankheiten und insbesondere von dissiminierter Lupus erythematodes.

14. Komplexer Vektor nach einem beliebigen der Ansprüche 6 bis 10 für seine Anwendung zur Behandlung von Autoimmunkrankheiten und insbesondere von dissimirtierter Lupus ery thematodes.
